# EUROPEAN PATENT APPLICATION

(11) **EP 1 217 568 A2**
(43) Date of publication of application: **26.06.2002**
(21) Application number: 01126048.6
(22) Date of filing: 31.10.2001
(51) Int. Cl.: G06F 19/00

(54) **Method, system and program for searching fragments from data sequences**

(30) Priority: 21.12.2000 US 741078
(71) Applicant: INDUSTRIAL TECHNOLOGY RESEARCH INSTITUTE, Chutung, Hsinchu (TW)
(72) Inventor: Yao, Wen-Hsuang, c/o Ind. Techn. Research Inst., Chutung, Hsinchu (TW)
(74) Representative: Rupp, Christian, Dipl.Phys.

(57) **Abstract**

The present invention provides a novel method, system, and computer program of searching for a pair of fragments from two data sequences, and in particular provides a method of searching for a pair of fragments from two biological sequences, such as nucleotide sequences and amino acid sequences. First of all, a pair of fragments from two data sequences are selected for their certain data arrangement. The next pair of fragments are extended in the same direction. If this data arrangement thereof satisfies a user-defined condition, the next fragments are further extended continuously. On the other hand, if the data arrangement does not satisfy the condition, it is then to match the fragments by gap insertion. After gap insertion, if the data arrangement becomes satisfies the condition, then steps continues to the next fragments extension. Otherwise the extension is terminated and the resulted fragments are obtained.

## Description

### FIELD OF THE INVENTION

The present invention relates to a method, system, and computer program of searching for a pair of fragments from two data sequences, wherein the data sequences preferably comprise biological sequences, such as nucleotide sequences and amino acid sequences.

### BACKGROUND OF THE INVENTION

There are many conventional techniques for sequence search. Some of these conventional search techniques include Needlemann-Wunsch or the original Wilbur-Lipman algorithms, FASTA, FASTP, and BLAST.

The Needlemann-Wunsch algorithm is a dynamic programming technique. This is an expensive technique since the amount of computation required is proportional to the length of two sequences to be compared. Because of the computational requirements, dynamic programming algorithms are impractical for searching large database or matching long data sequences, such as genome data, without the use of a supercomputer or other special purpose hardware. The method also has disadvantages in terms of search time and arbitrary result.

For this reason, faster heuristic methods (BLAST, FASTA ) are far more popular than rigorous dynamic programming. However, the penalty of using a heuristic method is the potential loss of accuracy. In the case of BLAST, the algorithm does an in-depth comparison of the original sequence and reference sequence only if they satisfy an initial minimal similarity test which can be performed very quickly. This reduces the amount of computation at risk of missing some matches that does not satisfy the initial criteria. About 20% of the similarities detected with the Needleman-Wunch algorithm are not picked up by BLAST.

Accordingly, there remains a need in the art for an improved method in both speed and sensitivity for comparing two long data sequences, especially huge genome sequences.

### SUMMARY OF THE INVNETION

Therefore, the problems stated above and related drawbacks of the prior art are solved with the principles of the present invention. In one aspect, the invention provides a method to search for a pair of fragments (Fx and Fy) from two data sequences (X and Y). The method comprises: **[1]** selecting a first fragment (Fx1) of X and a first fragment (Fy1) of Y, wherein a first pattern (p1) of Fx1 and Fy1 satisfies a first predetermined condition (c1); **[2]** extending a second fragment (Fx2) from Fx1 in a direction and extending a second fragment (Fy2) from Fy1 in the direction; **[3]** identifying a second pattern (p2) of Fx2 and Fy2; and **[4]** obtaining the pair of fragments (Fx and Fy) based on p2 in a predetermined manner.

Other aspects and advantages of the present invention will become apparent from the following detailed description and the examples.

### BRIEF DESCRIPTION OF THE DRAWINGS

The present invention will become fully understood from the detailed description given herein below with the accompanying drawings, given by way of illustration only and thus not intended to be limitative of the present invention.

**FIG.1** is a flow chart indication the framework of the invention.

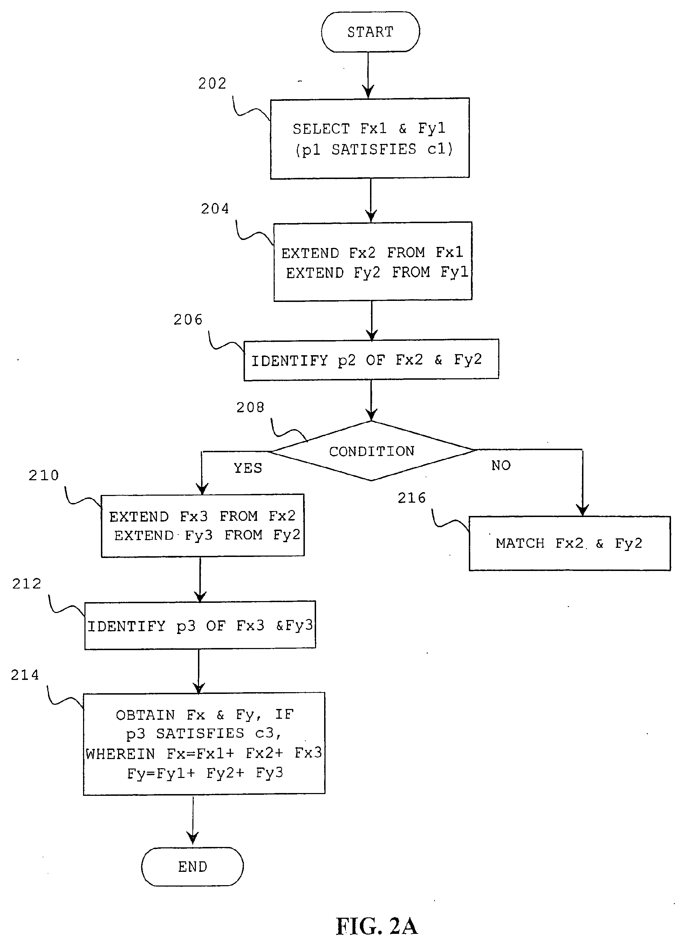

**FIG.2A** is a flow chart of one preferred embodiment of the invention.

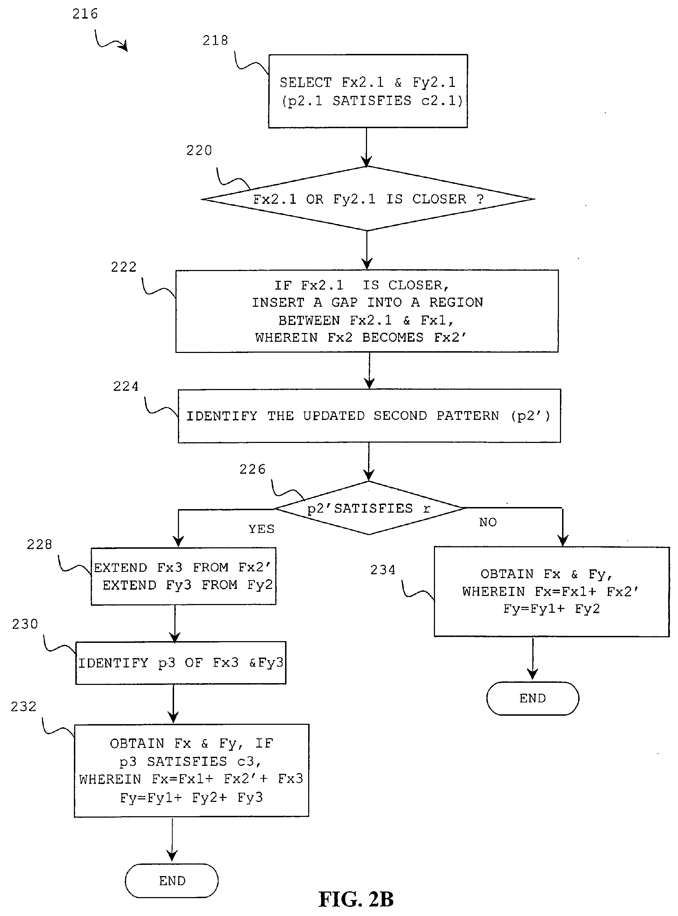

**FIG.2B** illustrates the step ***216*** of FIG. 2A in detail.

**FIG.3,** comprising FIG.3A, 3B, 3C, 3D, and 3E, is a flow chart of the most preferred embodiment of the invention.

FIG.4 shows the relationship between false positive and sensitivity in each database search result.

### DETAILED DESCRIPTION OF THE PRESENT INVENTION

One aspect of the present invention is used to provide a method of searching for a pair of fragments from two data sequences.

To describe the invention clearly, a number of definitions of terms used herein are given as follows.

The term "data sequence" used herein refers to a 1-dimension data sequence, including but are not be limited to a biological sequence, such as a nucleic acid sequence and amino acid sequences, a voiceprint, a electric wave.

The term "data" used herein refers to data included in the data sequence. For example, data in a nucleic acid sequence mean a nucleotide or nucleotides, data in a protein sequence mean an amino acid or amino acids, and data in a voiceprint may represent voice amplitude.

The term "fragment" used herein refers to a part of data sequences. For a DNA sequence with 1000 nucleotides, a part of the sequence, for example, from nucleotide 1 to nucleotide 100 represents a 'fragment' thereof. The length of the fragment can be defined by users in accordance with different application or need.

The term a "pattern" of two data sequences used herein refers to the data arrangement between two data sequences. The pattern can be shown as, for example, a number of identical bases or a similarity percentage between two DNA sequences.

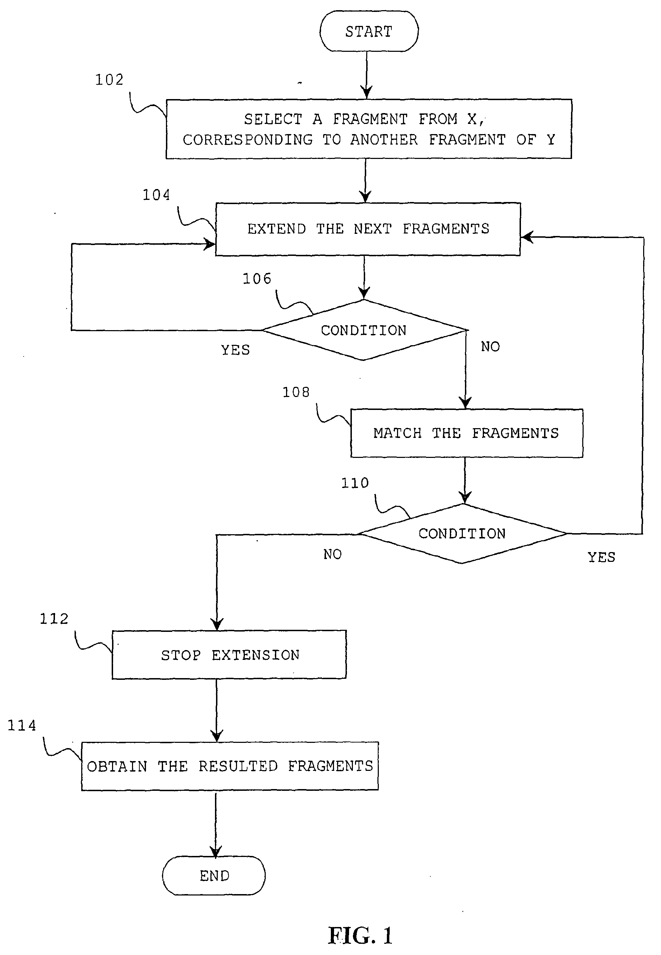

The flow chart of the framework of the invention is shown as the block diagram of **FIG.1**. There are two data sequences (X and Y). In one aspect of the invention, the data sequences are biological sequences (e.g. nucleotide sequences or amino acid sequences). The method starts by selecting a fragment from X, which corresponds to another fragment of Y ***102*** (e.g. the fragments are selected with the same length and same data information.). The next fragments are extended in the same direction ***104.*** It is then determined if the next fragments satisfy a given condition ***106.*** If the condition in step ***106*** is satisfied, operation goes back to step ***104*** to proceed the next extension. If the condition is not satisfied, then operation continues to step ***108.*** In step ***108,*** the extended fragments are matched to adjust the status thereof. Then it is determined if the fragments satisfy a given condition ***110.*** The condition in step ***106*** may be the same as that in step ***110,*** which depends on the need of users. If the condition in step ***110*** is satisfied, then operation goes back to step ***104*** to continue the next extension. If the condition in step ***110*** is not satisfied, the next extension is terminated. Thus a resulted pair of fragments are obtained.

To further describe the invention clearly, more definitions of terms used herein are given. The term "condition", "criterion" and "requirement" used herein mean a status, degree, or level between sequences or fragments, which can be defined by users according to their needs. With reference to the definition of "pattern" stated above, when one says a "pattern" of two fragments satisfies a "condition", it means that an identical percentage between the two fragments is above 50%, for example.

In particular, the method of the invention starts by selecting a first fragment (Fx1) of X and a first fragment (Fy1) of Y, wherein a first pattern (p1) of Fx1 and Fy1 satisfies a first predetermined condition (c1) ***202.*** More preferably, Fx1 and Fy1 are of the same length and have a highly identical percentage. Next, two of the second fragments (Fx2 and Fy2) are extended from Fx1 and Fy1 in the same direction ***204,*** for example, left or right. Preferably, fragments can be extended simultaneously in both directions. After the extension, a second pattern (p2) of Fx2 and Fy2 is identified ***206.*** It is then determined if p2 satisfy a second predetermined condition (c2) ***208.*** If p2 satisfies the condition (c2), operation continues to step ***210.*** On the other hand, if p2 does not satisfy the condition (c2), operation continues to step ***216,*** wherein it is to match Fx2 and Fy2 to optimize the status thereof. In step ***210,*** two of the third fragments (Fx3 and Fy3) are extended from Fx2 and Fy2 in the direction ***210.*** After that, a third pattern (p3) of Fx3 and Fy3 is identified ***212.*** If p3 satisfies a third predetermined condition (c3), then a resulted pair of fragments (Fx and Fy) is obtained ***214,*** wherein Fx includes Fx1, Fx2, and Fx3, and Fy includes Fy1, Fy2, and Fy3. For example, when either Fx3 or Fy3 encounters the end of X or Y, the extension is stopped, and the resulted fragments as described above are obtained.

To describe the below invention clearly, there is still a need to provide a number of definitions of terms used herein. The term "subfragment" used herein means a part of fragment. For a fragment of 100 nucleotides, a length of the sequence, for example, from nucleotide 23 to nucleotide 30 means a 'subfragment' thereof. Users can define the suitable length of the subfragments in accordance with their needs.

The term "subpattern" used herein means the data arrangement of two subfragments. The subpattern can be shown by the way similar to that of "pattern" described above, for example, as a number of identical bases or a similarity percentage for two DNA sequences.

The term "subcondition" used herein means a status, degree, or level of subfragments, which can be defined by users depending on their needs. A "subcondition" can be expressed by the way similar to that of "condition".

The term "gap" used herein means a number of spaces or meaningless data, which is typically shown as a "dash" ("-"). For example, a gap of 4 meaningless data can be shown as "- - - -".

The term " to match Fx2 and Fy2" used herein means to optimize the data arrangement between Fx2 and Fy2 by gap insertion within a suitable region of X or Y data sequence.

To carry out step ***216,*** two subfragments (Fx2.1 and Fy2.1) are selected ***218,*** wherein a subpattem (p2.1) of Fx2.1 and Fy2.1 satisfies a subcondition (c2.1) which can be defined by users. It is preferred to determine that whether Fx2.1 or Fy2.1 is geographically closer to the pair of Fx1 and Fy1 ***220.*** If Fx2.1 is closer, a suitable gap is inserted into a region between Fx2.1 and Fx1 ***222*** such that a part of data in Fx2 are excluded therefrom, whereby Fx2 becomes an updated fragment, designated as Fx2', which includes the gap but excludes the part of data in Fx2. After the insertion step, an updated second pattern, named as p2', of Fx2' and Fy2, is generated and identified ***224.*** Next, it is determined if p2' satisfies a requirement (r) ***226.*** If the requirement (r) in step ***226*** is satisfied, then operation continues to step ***228.*** On the other hand, if p2' does not satisfy the requirement (r), operation continues to step ***234***.

In step ***228,*** two of the third fragments (Fx3 and Fy3) are extended from Fx2' and Fy2 in the direction ***228.*** After that, a third pattern (p3) of Fx3 and Fy3 is identified ***230.*** If p3 satisfies a third predetermined condition (c3), then a resulted pair of fragments (Fx and Fy) is obtained ***232,*** wherein Fx includes Fx1, Fx2', and Fx3, and Fy includes Fy1, Fy2, and Fy3 as well. For example, when either Fx3 or Fy3 encounters the end of X or Y, the extension is stopped, and thus the resulted fragments mentioned above are obtained.

In step ***234***, when p2'does not satisfy the requirement (r), a resulted pair of fragments (Fx and Fy) are obtained, wherein Fx includes Fx1 and Fx2', and Fy includes Fy1 and Fy2.

Those skilled in the art will appreciate that a gap will be inserted into a region between Fy2.1 and Fy1 to optimize the pattern of Fx2 and Fy2 if the distance between Fy2.1 ad Fy1 is closer than that between Fx2.1 and Fx1. In this case, those skilled in the art will thus readily obtain a resulted pair of fragments with reference to steps from ***222*** to ***234****.*

In another aspect of the present invention, there is still a system provided searching for a pair of fragments from two data sequences.

The system comprises **[1]** logic that selects a first fragment (Fx1) of X and a first fragment (Fy1) of Y, wherein a first pattern (p1) of Fx1 and Fy1 satisfies a first predetermined condition (c1), **[2]** logic that extends a second fragment (Fx2) from Fx1 and a second fragment (Fy2) from Fy1 in the same direction, **[3]** logic that identifies a second pattern (p2) of Fx2 and Fy2, and **[4]** logic that obtains the resulted pair of fragments (Fx and Fy) based on p2 by a predetermined logic.

In one preferred embodiment of the invention, the data sequences are biological sequences (e.g. nucleotide sequences or amino acid sequences).

The predetermined logic further comprises logic that determines if p2 satisfies a user-defined condition (c2), logic that extends a third fragment (Fx3) from Fx2 and a third fragment (Fy3) from Fy2 in the direction if p2 satisfies the condition c2, and logic that matches Fx2 and Fy2 if p2 does not satisfy c2.

The logic that extends a third fragment (Fx3) from Fx2 and a third fragment (Fy3) from Fy2 in the direction, when p2 satisfies c2, still comprises logic that identifies a third pattern (p3) of Fx3 and Fy3, logic that determines if p3 satisfies a user-defined condition (c3), and logic that obtains the resulted pair of fragments (Fx and Fy) if p3 satisfies c3, wherein Fx includes Fx1, Fx2, and Fx3, and Fy includes Fy1, Fy2, and Fy3.

The logic mentioned above that matches Fx2 and Fy2 still comprises logic that selects a subfragment (Fx2.1) in FX2 and a subfragment (Fy2.1) in FY2, in which a subpattern (p2.1) of Fx2.1 and Fy2.1 satisfies a subcondition, logic that inserts a gap into a region between Fx2.1 and Fx1 to exclude a part of data in Fx2 therefrom, whereby Fx2 becomes an updated fragment (Fx2') which includes the gap but excludes the part of data in Fx2, logic that identifies an updated p2 (p2') between Fx2' and Fy2, logic that determines if p2' satisfies a user-defined requirement (r), logic that extends a third fragment (Fx3) from Fx2' and a third fragment (Fy3) from Fy2 in the direction if p2' satisfies r, wherein Fx3 includes the part of data in Fx2, and logic that obtains the resulted pair of fragments (Fx and Fy) if p2' does not satisfy r, wherein Fx includes Fx1 and Fx2', and Fy includes Fy1 and Fy2.

The logic that extends a third fragment (Fx3) from Fx2' and a third fragment (Fy3) from Fy2 in the direction still comprises logic that identifies a third pattern (p3) of Fx3 and Fy3, logic that determines if p3 satisfies a user-defined condition (c3), and logic that obtains the resulted pair of fragments (Fx and Fy) if p3 satisfies c3, wherein Fx includes Fx1, Fx2' and Fx3, and Fy includes Fy1, Fy2 and Fy3.

In another aspect of the present invention, there is still provided a computer program of searching for a pair of fragments from two data sequences.

The computer program comprises: **[1]** a code segment that selects a first fragment (Fx1) of X and a first fragment (Fy1) of Y, wherein a first pattern (p1) of Fx1 and Fy1 satisfies a user-defined condition (c1), **[2]** a code segment that extends a second fragment (Fx2) from Fx1 and a second fragment (Fy2) from Fy1 in the same direction, **[3]** a code segment that identifies a second pattern (p2) of Fx2 and Fy2, and **[4]** a code segment that obtains the resulted fragments (Fx and Fy) based on p2 by a predetermined code segment.

In one preferred embodiment of the invention, the data sequences described above are biological sequences (e.g. nucleotide sequences or amino acid sequences).

The predetermined code segment still comprises a code segment that determines if p2 satisfies a user-defined condition (c2), a code segment that extends a third fragment (Fx3) from Fx2 and a third fragment (Fy3) from Fy2 in the direction if p2 satisfies c2, and a code segment that matches Fx2 and Fy2 if p2 does not satisfy c2.

The code segment that extends a third fragment (Fx3) from Fx2 and a third fragment (Fy3) from Fy2 in the direction if p2 satisfies c2, still comprises a code segment that identifies a third pattern (p3) of Fx3 and Fy3, a code segment that determines if p3 satisfies a third predetermined condition (c3), and a code segment that obtains the resulted fragments (Fx and Fy) if p3 satisfies c3, wherein Fx includes Fx1, Fx2 and Fx3, and Fy includes Fy1, Fy2 and Fy3.

The code segment mentioned above that matches Fx2 and Fy2 still comprises a code segment that selects a subfragment (Fx2.1) in FX2 and a subfragment (Fy2.1) in FY2, wherein a subpattern (p2.1) of Fx2.1 and Fy2.1 satisfies a user-defined subcondition, a code segment that inserts a gap into a region between Fx2.1 and Fx1 to exclude a part of data in Fx2 therefrom, whereby Fx2 becomes an updated fragment (Fx2') which includes the gap, but the part of data in Fx2 is excluded, a code segment that identifies an updated p2 (p2') between Fx2' and Fy2, a code segment that determining if p2' satisfies a user-defined requirement (r), a code segment that extends a third fragment (Fx3) from Fx2' and a third fragment (Fy3) from Fy2 in the direction if p2' satisfies r, wherein Fx3 includes the part of data in Fx2, and a code segment that obtains the resulted fragments (Fx and Fy) if p2' does not satisfy r, wherein Fx includes Fx1 and Fx2', and Fy includes Fy1 and Fy2.

The code segment that extends a third fragment (Fx3) from Fx2' and a third fragment (Fy3) from Fy2 in the direction comprises a code segment that identifies a third pattern (p3) of Fx3 and Fy3, a code segment that determines if p3 satisfies a user-defined condition (c3), and a code segment that obtains the resulted fragments (Fx and Fy) if p3 satisfies c3, wherein Fx includes Fx1, Fx2' and Fx3, and Fy includes Fy1, Fy2 and Fy3.

### Example 1

The present invention will become apparent with reference to the below examples. Those examples are given by way of illustration only and thus not intended to be any limitation of the present invention.

The most preferred embodiment of the present invention has specific application to search the genome of living organisms, in particular human genome, to find biological information therefrom, such as the fragment of interesting sequences.

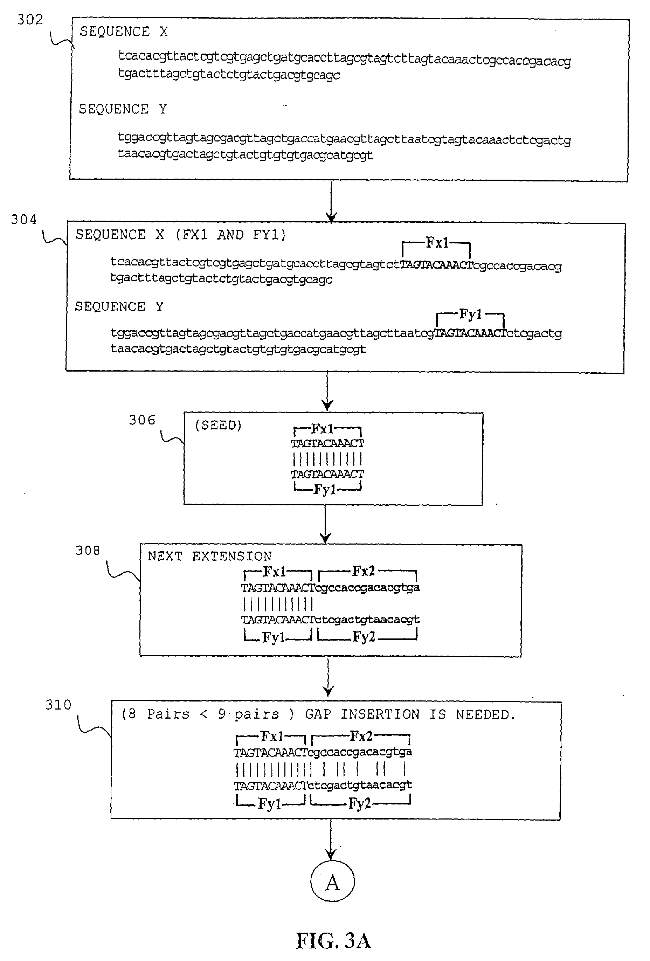

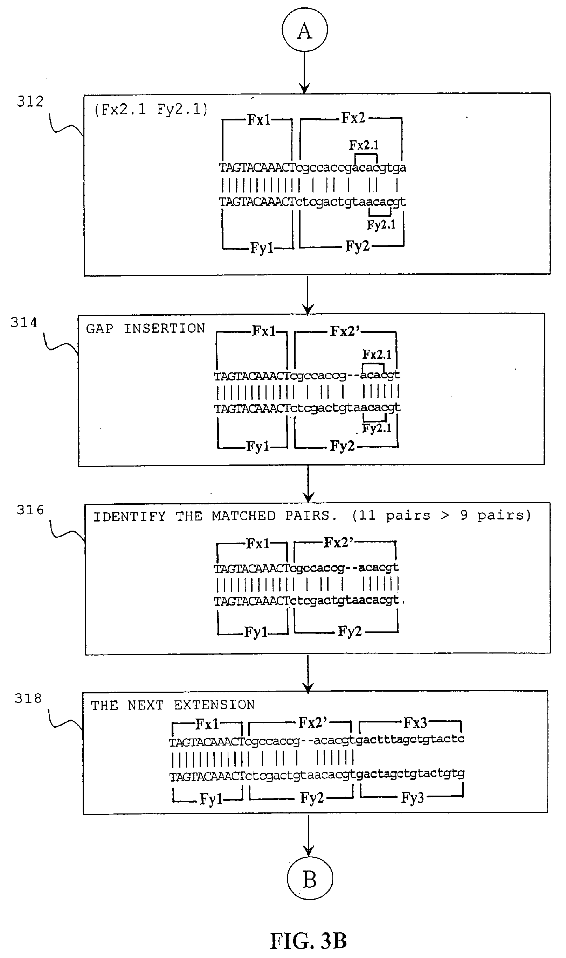

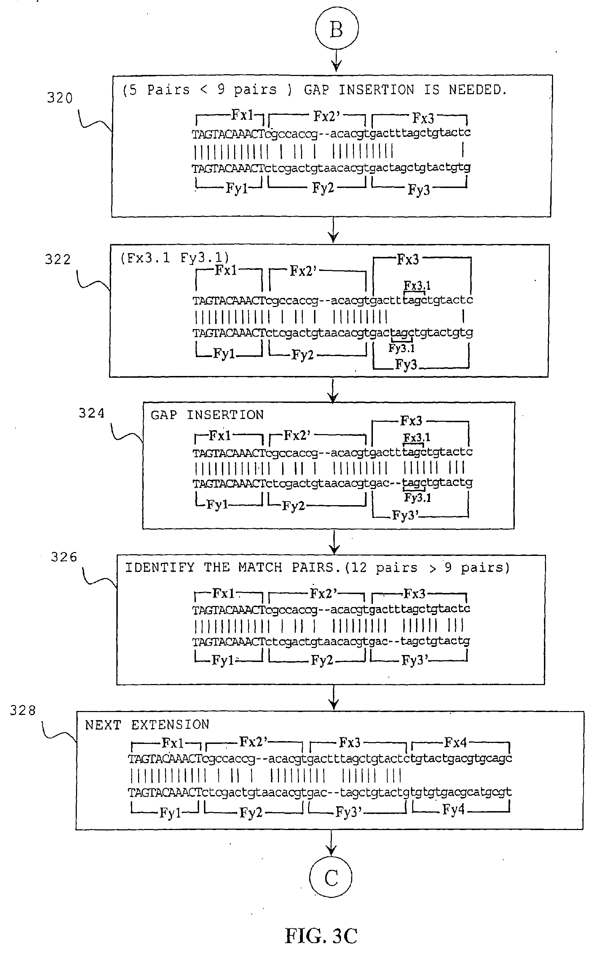

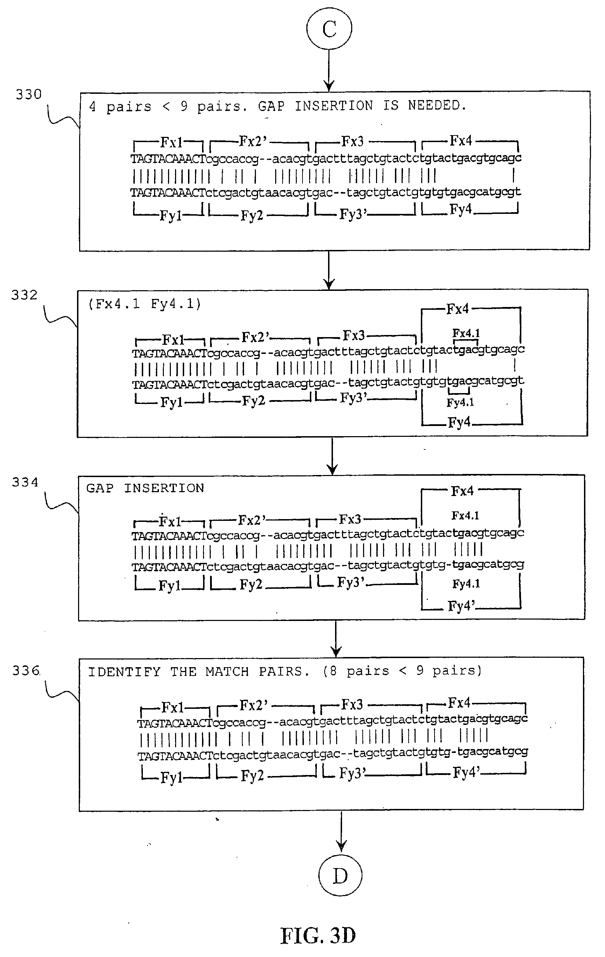

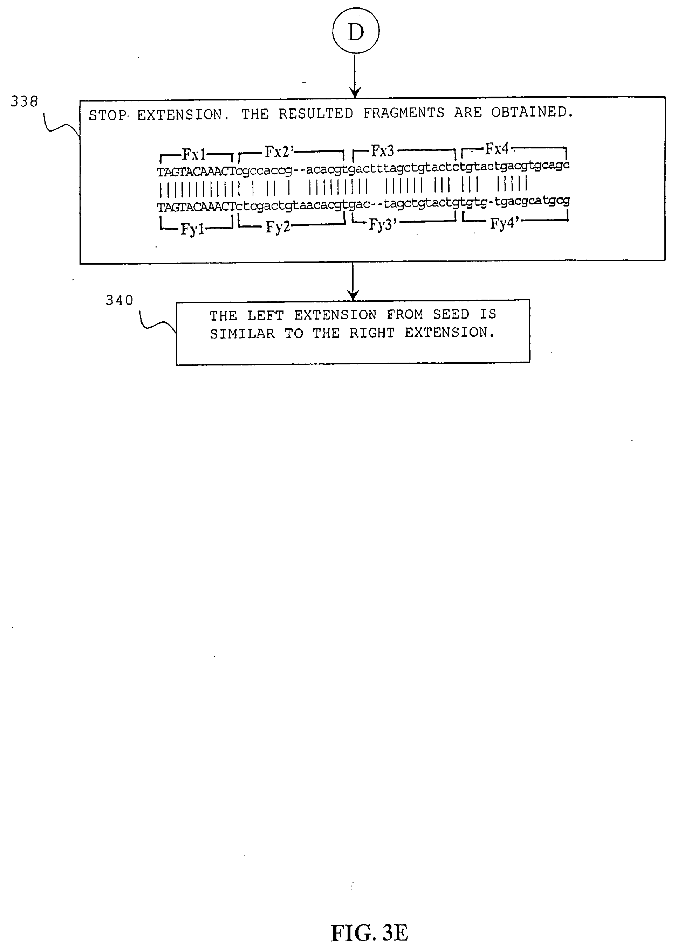

With reference to FIG 3, there are two DNA sequences to be compared, designated as X and Y ***302.*** Two identical fragments (Fx1 and Fy1) are selected ***304,*** wherein they are in the same length of 11 bases and the identical percentage is 100%. The pair of fragments, Fx1 and Fy1, typically named as a "seed" ***306,*** are extended in the right direction to create a next pair of fragments, Fx2 and Fy2 ***308.*** The numbers of matched sequences of Fx2 and Fy2 are detected as 8 pairs, which are lower than a user-defined criterion, 9 pairs ***310.*** Thus it is required to match Fx2 and Fy2 in a manner as set forth before. The match procedure starts by selecting two subfragments (Fx2.1 and Fy2.1) respectively from Fx2 and Fy2 ***312,*** wherein the two subfragments have the same length and the same nucleotide sequences, "acac", for example. A gap is inserted into the position in front of the first base of Fx2.1 ***314*** such that the nucleotide sequences of Fx2 are shifted, and Fx2 becomes an updated fragment, named as Fx2'. The match number is increased to be 11 pairs ***316,*** which is greater than the user-defined criterion of 9 pairs. Therefore the next fragments (Fx3 and Fx3) are extended subsequently ***318.***

The number of matched base pairs of Fx3 and Fy3 are detected as 5 pairs ***320,*** which is lower than 9 pairs. Then the match procedure starts as set forth herein before. Two identical subfragments (Fx3.1 and Fy3.1) are selected ***322.*** A suitable gap is inserted into the position in front of the first base of Fy3.1 ***324*** such that Fy3 becomes an updated fragment, named as Fy3'. The match number of Fy3' and Fx3 becomes 12 pairs, which is larger than 9 pairs ***326.*** Therefore the next fragments (Fx4 and Fy4) are extended subsequently ***328.***

The number of matched base pairs of Fx4 and Fy4 are detected as 4 pairs, which is lower than 9 pairs ***330.*** Therefore the match procedure starts as said before. Two identical subfragments (Fx4.1 and Fy4.1) are selected ***332.*** A suitable gap is inserted ***334*** such that Fy4 becomes an updated fragment, named as Fy4'. The match number of Fy4' and Fx4 turns to be 8 pairs, which is still lower than 9 pairs ***336.*** Therefore the next extension is stopped. The resulted fragments are obtained, which are Fx1+Fx2'+Fx3+Fx4 and Fy1+Fy2+Fy3'+Fy4' ***338.***

Since the matched numbers of Fy4' and Fx4 can not reach the criterion by the way of gap insertion, it is suggested that the subsequent fragments after Fy4' and Fx4 may hardly satisfy the criterion, either. That's the reason for stopping the next extension. In the case, users can optionally process the last extended fragments, Fy4' and Fx4, to modify the end of the fragments resulted from the invention. For example, one can keep the front pairs "tgt" but give up the remainders of Fy4' and Fx4. There are various ways for processing the last extended fragment. Those skilled in the art will appreciate those processing method, and therefore the scope of the invention should not be limited by the processing method.

Those skilled in the art will be aware that the left extension from Fx1 and Fy1 can be performed with the same principle ***340*** as that of the right extension described above.

To show the advantages of the invention, database search for a query sequence in a certain length were performed by conventional method and the method of the present invention.

All tests were conducted on a 500 MHz Pentium III computer equipped with 1024 MB of RAM. The average CPU time required in the method of the invention (FLAG) to execute database search for a query sequence of a certain length was shown in Table 1, with standard error shown in parentheses.

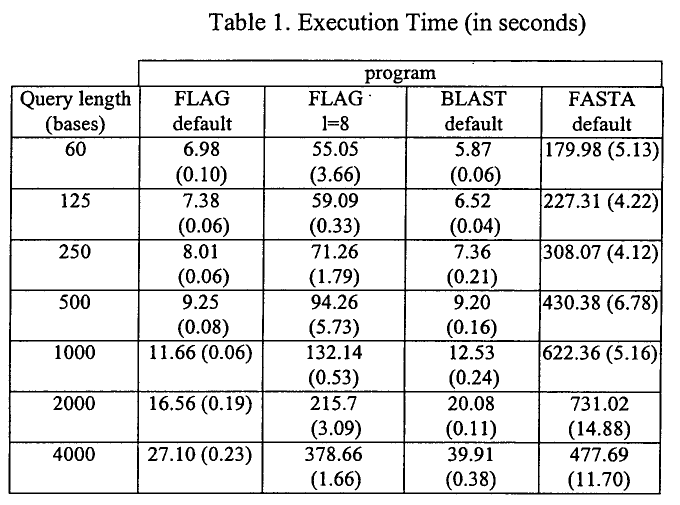

The different lengths used were 60, 125, 250, 500, 1000, 2000, and 4000 bases, respectively. "FLAG default" was the CPU time for FLAG with default setting. "FLAG I = 8" was the CPU time for FLAG with a seed size of 8. The execution time needed by BLAST and FASTA (both with default setting) was shown in the last two columns.

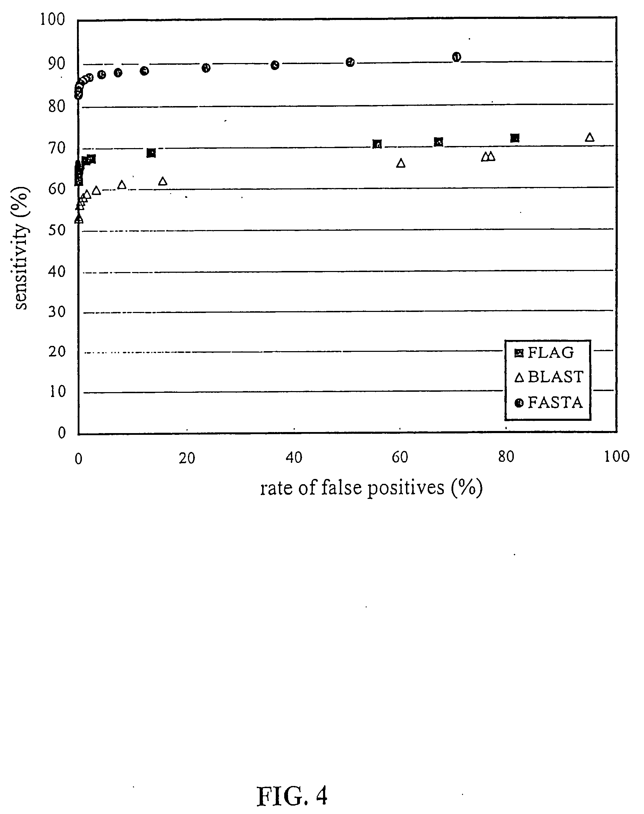

In a similar test, the relationship between the rate of false positives and sensitivity for different search method was shown in FIG. 4. "False positives" were the sequences satisfied a user-defined condition thus was listed in the search result but in fact they were unrelated to the query sequence. "Sensitivity" was a reference to evaluate the performance of the method used herein. Typically, a higher sensitivity resulted in a higher performance.

As shown in Table 1 and FIG.4, the execution time, in the same query length, needed by the method of the present invention was reduced about 30 to 50 folds than that of FASTA needed, although FASTA has higher sensitivity percentage. In addition, the method provided by the present invention has improved performance in both the execution time and sensitivity percentage, when comparing to the most conventional search method, i.e. BLAST. Accordingly, The invention indeed provides a valuable search method with comparable sensitivity, particularly in the field of genome search, to solve the problems in the conventional method.

### Example 2

The method of the present invention can also be applied to determining the similarity between two sequences. Using the method herein, those skilled in the art are able to determine the similarity of the resulted pair of fragments based on each pair of extended fragments in a conventional manner, such as statistics.

### Example 3

Voice recognition is also a potential application using the present invention. A song or a speech is a sequence of notes or phonemes. They are also 1-dimentional data sequences. Therefore, the method of the invention can be used to compare or to determine the identities between two note sequences or two phoneme sequences.

While various embodiments have been described above, it should be understood that they have been presented by way of example only, but not limitation. Thus, the breadth and scope of a preferred embodiment should not be limited by any of the above described exemplary embodiments, but should be defined only in accordance with the following claims and their equivalents

## Claims

1. A method of searching for a pair of fragments (Fx and Fy) from two data sequences (X and Y), wherein a pattern of Fx and Fy satisfies a predetermined criterion, said method comprising the steps of:
(1.1) selecting a first fragment (Fx1) of X and a first fragment (Fy1) of Y, wherein a first pattern (p1) of Fx1 and Fy1 satisfies a first predetermined condition (c1);
(1.2) extending a second fragment (Fx2) from Fx1 in a direction and extending a second fragment (Fy2) from Fy1 in the direction;
(1.3) identifying a second pattern (p2) of Fx2 and Fy2; and
(1.4) obtaining the pair of fragments (Fx and Fy) based on p2 in a predetermined manner.

2. The method of claim 1, wherein said predetermined manner comprises the steps of:
(2.1) determining if p2 satisfies a second predetermined condition (c2);
(2.1.1) extending a third fragment (Fx3) from Fx2 in the direction and extending a third fragment (Fy3) from Fy2 in the direction if p2 satisfies c2; and
(2.1.2) matching Fx2 and Fy2 if p2 does not satisfy c2.

3. The method of claim 2, wherein the step (2.1.1) comprises the steps of:
(3.1) identifying a third pattern (p3) of Fx3 and Fy3;
(3.2) determining if p3 satisfies a third predetermined condition (c3); and
(3.3) obtaining the pair of fragments (Fx and Fy)if p3 satisfies c3, wherein Fx includes Fx1, Fx2, and Fx3, and Fy includes Fy1,Fy2, and Fy3.

4. The method of claim 2, wherein said step (2.1.2) of matching Fx2 and Fy2 comprises the steps of:
(4.1) selecting a subfragment (Fx2.1) in FX2 and a subfragment (Fy2.1) in FY2, wherein a subpattern (p2.1) of Fx2.1 and Fy2.1 satisfies a predetermined subcondition (c2.1);
(4.2) inserting a gap into a region between Fx2.1 and Fx1 to exclude a part of data in Fx2 therefrom, whereby Fx2 becomes an updated fragment (Fx2') which includes the gap, but the part of data in Fx2 is excluded;
(4.3) identifying an updated p2 (p2') between Fx2' and Fy2;
(4.4) determining if p2' satisfies a predetermined requirement (r);
(4.4.1) extending a third fragment (Fx3) from Fx2' in the direction and extending a third fragment (Fy3) from Fy2 in the direction if p2' satisfies r, wherein Fx3 includes the part of data in Fx2; and
(4.4.2) obtaining the pair of fragments (Fx and Fy) if p2' does not satisfy r, wherein Fx includes Fx1 and Fx2', and Fy includes Fy1 and Fy2.

5. The method of claim 4, wherein the step (4.4.1) comprises the steps of:
(5.1) identifying a third pattern (p3) of Fx3 and Fy3;
(5.2) determining if p3 satisfies a third predetermined condition (c3); and
(5.3) obtaining the pair of fragments (Fx and Fy) if p3 satisfies c3, wherein Fx includes Fx1, Fx2' and Fx3, and Fy includes Fy1,Fy2 and Fy3.

6. A method of searching for a pair of fragments (Fx and Fy) from two biological sequences (X and Y), wherein a pattern of Fx and Fy satisfies a predetermined criterion, said method comprising the steps of:
(6.1) selecting a first fragment (Fx1) of X and a first fragment (Fy1) of Y, wherein a first pattern (p1) of Fx1 and Fy1 satisfies a first predetermined condition (c1);
(6.2) extending a second fragment (Fx2) from Fx1 in a direction and extending a second fragment (Fy2) from Fy1 in the direction;
(6.3) identifying a second pattern (p2) of Fx2 and Fy2; and
(6.4) obtaining the pair of fragments (Fx and Fy) based on p2 in a predetermined manner.

7. The method of claim 6, wherein said predetermined manner comprises the steps of:
(7.1) determining if p2 satisfies a second predetermined condition (c2);
(7.1.1) extending a third fragment (Fx3) from Fx2 in the direction and extending a third fragment (Fy3) from Fy2 in the direction if p2 satisfies c2; and
(7.1.2) matching Fx2 and Fy2 if p2 does not satisfy c2.

8. The method of claim 7, wherein the step (7.1.1) comprises the steps of:
(8.1) identifying a third pattern (p3) of Fx3 and Fy3;
(8.2) determining if p3 satisfies a third predetermined condition (c3); and
(8.3) obtaining the pair of fragments (Fx and Fy) if p3 satisfies c3, wherein Fx includes Fx1, Fx2, and Fx3, and Fy includes Fy1, Fy2, and Fy3.

9. The method of claim 7, wherein said step (7.1.2) of matching Fx2 and Fy2 comprises the steps of:
(9.1) selecting a subfragment (Fx2.1) in FX2 and a subfragment (Fy2.1) in FY2, wherein a subpattern (p2.1) of Fx2.1 and Fy2.1 satisfies a predetermined subcondition (c2.1);
(9.2) inserting a gap into a region between Fx2.1 and Fx1 to exclude a part of biological data in Fx2 therefrom, whereby Fx2 becomes an updated fragment (Fx2') which includes the gap, but the part of biological data in Fx2 is excluded;
(9.3) identifying an updated p2 (p2') between Fx2' and Fy2;
(9.4) determining if p2' satisfies a predetermined requirement (r);
(9.4.1) extending a third fragment (Fx3) from Fx2' in the direction and extending a third fragment (Fy3) from Fy2 in the direction if p2' satisfies r, wherein Fx3 includes the part of biological data in Fx2; and
(9.4.2) obtaining the pair of fragments (Fx and Fy) if p2' does not satisfy r, wherein Fx includes Fx1 and Fx2', and Fy includes Fy1 and Fy2.

10. The method of claim 9, wherein the step (9.4.1) comprises the steps of:
(10.1) identifying a third pattern (p3) of Fx3 and Fy3;
(10.2) determining if p3 satisfies a third predetermined condition (c3); and
(10.3) obtaining the pair of fragments (Fx and Fy) if p3 satisfies c3, wherein Fx includes Fx1, Fx2' and Fx3, and Fy includes Fy1,Fy2 and Fy3.

11. A system of searching for a pair of fragments (Fx and Fy) from two data sequences (X and Y), wherein a pattern of Fx and Fy satisfies a predetermined criterion, said system comprising:
(11.1) logic that selects a first fragment (Fx1) of X and a first fragment (Fy1) of Y, wherein a first pattern (p1) of Fx1 and Fy1 satisfies a first predetermined condition (c1);
(11.2) logic that extends a second fragment (Fx2) from Fx1 in a direction and extends a second fragment (Fy2) from Fy1 in the direction;
(11.3) logic that identifies a second pattern (p2) of Fx2 and Fy2; and
(11.4) logic that obtains the pair of fragments (Fx and Fy) based on p2 by a predetermined logic.

12. The system of claim 11, wherein said predetermined logic comprises
(12.1) logic that determines if p2 satisfies a second predetermined condition (c2);
(12.1.1) logic that extends a third fragment (Fx3) from Fx2 in the direction and extending a third fragment (Fy3) from Fy2 in the direction if p2 satisfies c2; and
(12.1.2) logic that matches Fx2 and Fy2 if p2 does not satisfy c2.

13. The system of claim 12, wherein the logic (12.1.1) comprises
(13.1) logic that identifies a third pattern (p3) of Fx3 and Fy3;
(13.2) logic that determines if p3 satisfies a third predetermined condition (c3); and
(13.3) logic that obtains the pair of fragments (Fx and Fy) if p3 satisfies c3, wherein Fx includes Fx1, Fx2 and Fx3, and Fy includes Fy1,Fy2 and Fy3.

14. The system of claim 12, wherein said logic (12.1.2) that matches Fx2 and Fy2 comprises:
(14.1) logic that selects a subfragment (Fx2.1) in FX2 and a subfragment (Fy2.1) in FY2, wherein a subpattern (p2.1) of Fx2.1 and Fy2.1 satisfies a predetermined subcondition (c2.1);
(14.2) logic that inserts a gap into a region between Fx2.1 and Fx1 to exclude a part of data in Fx2 therefrom, whereby Fx2 becomes an updated fragment (Fx2') which includes the gap, but the part of data in Fx2 is excluded;
(14.3) logic that identifies an updated p2 (p2') between Fx2' and Fy2;
(14.4) logic that determines if p2' satisfies a predetermined requirement (r);
(14.4.1) logic that extends a third fragment (Fx3) from Fx2' in the direction and extending a third fragment (Fy3) from Fy2 in the direction if p2' satisfies r, wherein Fx3 includes the part of data in Fx2; and
(14.4.2) logic that obtains the pair of fragments (Fx and Fy) if p2' does not satisfy r, wherein Fx includes Fx1 and Fx2', and Fy includes Fy1 and Fy2.

15. The system of claim 14, wherein the logic (14.4.1) comprises:
(15.1) logic that identifies a third pattern (p3) of Fx3 and Fy3;
(15.2) logic that determines if p3 satisfies a third predetermined condition (c3); and
(15.3) logic that obtains the pair of fragments (Fx and Fy) if p3 satisfies c3, wherein Fx includes Fx1, Fx2', and Fx3, and Fy includes Fy1,Fy2, and Fy3.

16. A system of searching for a pair of fragments (Fx and Fy) from two biological sequences (X and Y), wherein a pattern of Fx and Fy satisfies a predetermined criterion, said system comprises:
(16.1) logic that selects a first fragment (Fx1) of X and a first fragment (Fy1) of Y, wherein a first pattern (p1) of Fx1 and Fy1 satisfies a first predetermined condition (c1);
(16.2) logic that extends a second fragment (Fx2) from Fx1 in a direction and extending a second fragment (Fy2) from Fy1 in the direction;
(16.3) logic that identifies a second pattern (p2) of Fx2 and Fy2; and
(16.4) logic that obtains the pair of fragments (Fx and Fy) based on p2 by a predetermined logic.

17. The system of claim 16, wherein said predetermined logic comprises:
(17.1) logic that determines if p2 satisfies a second predetermined condition (c2);
(17.1.1) logic that extends a third fragment (Fx3) from Fx2 in the direction and extending a third fragment (Fy3) from Fy2 in the direction if p2 satisfies c2; and
(17.1.2) logic that matches Fx2 and Fy2 if p2 does not satisfy c2.

18. The system of claim 17, wherein the logic (17.1.1) comprises:
(18.1) logic that identifies a third pattern (p3) of Fx3 and Fy3;
(18.2) logic that determines if p3 satisfies a third predetermined condition (c3); and
(18.3) logic that obtains the pair of fragments (Fx and Fy) if p3 satisfies c3, wherein Fx includes Fx1, Fx2, and Fx3, and Fy includes Fy1, Fy2, and Fy3.

19. The system of claim 17, wherein said logic (17.1.2) of matching Fx2 and Fy2 comprises:
(19.1) logic that selects a subfragment (Fx2.1) in FX2 and a subfragment (Fy2.1) in FY2, wherein a subpattern (p2.1) of Fx2.1 and Fy2.1 satisfies a predetermined subcondition (c2.1);
(19.2) logic that inserts a gap into a region between Fx2.1 and Fx1 to exclude a part of biological data in Fx2 therefrom, whereby Fx2 becomes an updated fragment (Fx2') which includes the gap, but the part of biological data in Fx2 is excluded;
(19.3) logic that identifies an updated p2 (p2') between Fx2' and Fy2;
(19.4) logic that determines if p2' satisfies a predetermined requirement (r);
(19.4.1) logic that extends a third fragment (Fx3) from Fx2' in the direction and extending a third fragment (Fy3) from Fy2 in the direction if p2' satisfies r, wherein Fx3 includes the part of biological data in Fx2; and
(19.4.2) logic that obtains the pair of fragments (Fx and Fy) if p2' does not satisfy r, wherein Fx includes Fx1 and Fx2', and Fy includes Fy1 and Fy2.

20. The system of claim 19, wherein the logic (19.4.1) comprises
(20.1) logic that identifies a third pattern (p3) of Fx3 and Fy3;
(20.2) logic that determines if p3 satisfies a third predetermined condition (c3); and
(20.3) logic that obtains the pair of fragments (Fx and Fy) if p3 satisfies c3, wherein Fx includes Fx1, Fx2' and Fx3, and Fy includes Fy1,Fy2 and Fy3.

21. A computer program of searching for a pair of fragments (Fx and Fy) from two data sequences (X and Y), wherein a pattern of Fx and Fy satisfies a predetermined criterion, said computer program comprising :
(21.1) a code segment that selects a first fragment (Fx1) of X and a first fragment (Fy1) of Y, wherein a first pattern (p1) of Fx1 and Fy1 satisfies a first predetermined condition (c1);
(21.2) a code segment that extends a second fragment (Fx2) from Fx1 in a direction and extends a second fragment (Fy2) from Fy1 in the direction;
(21.3) a code segment that identifies a second pattern (p2) of Fx2 and Fy2; and
(21.4) a code segment that obtains the pair of fragments (Fx and Fy) based on p2 by a predetermined code segment.

22. The computer program of claim 21, wherein said predetermined code segment comprises :
(22.1) a code segment that determines if p2 satisfies a second predetermined condition (c2);
(22.1.1) a code segment that extends a third fragment (Fx3) from Fx2 in the direction and extending a third fragment (Fy3) from Fy2 in the direction if p2 satisfies c2; and
(22.1.2) a code segment that matches Fx2 and Fy2 if p2 does not satisfy c2.

23. The computer program of claim 22, wherein the code segment (22.1.1) comprises
(23.1) a code segment that identifies a third pattern (p3) of Fx3 and Fy3;
(23.2) a code segment that determines if p3 satisfies a third predetermined condition (c3); and
(23.3) a code segment that obtains the pair of fragments (Fx and Fy) if p3 satisfies c3, wherein Fx includes Fx1, Fx2 and Fx3, and Fy includes Fy1,Fy2 and Fy3.

24. The computer program of claim 22, wherein said code segment (22.1.2) that matches Fx2 and Fy2 comprises:
(24.1) a code segment that selects a subfragment (Fx2.1) in FX2 and a subfragment (Fy2.1) in FY2, wherein a subpattern (p2.1) of Fx2.1 and Fy2.1 satisfies a predetermined subcondition (c2.1);
(24.2) a code segment that inserts a gap into a region between Fx2.1 and Fx1 to exclude a part of data in Fx2 therefrom, whereby Fx2 becomes an updated fragment (Fx2') which includes the gap, but the part of data in Fx2 is excluded;
(24.3) a code segment that identifies an updated p2 (p2') between Fx2' and Fy2;
(24.4) a code segment that determining if p2' satisfies a predetermined requirement (r);
(24.4.1) a code segment that extends a third fragment (Fx3) from Fx2' in the direction and extending a third fragment (Fy3) from Fy2 in the direction if p2' satisfies r, wherein Fx3 includes the part of data in Fx2; and
(24.4.2) a code segment that obtains the pair of fragments (Fx and Fy) if p2' does not satisfy r, wherein Fx includes Fx1 and Fx2', and Fy includes Fy1 and Fy2.

25. The computer program of claim 24, wherein the code segment (24.4.1) comprises
(25.1) a code segment that identifies a third pattern (p3) of Fx3 and Fy3;
(25.2) a code segment that determines if p3 satisfies a third predetermined condition (c3); and
(25.3) a code segment that obtains the pair of fragments (Fx and Fy) if p3 satisfies c3, wherein Fx includes Fx1, Fx2' and Fx3, and Fy includes Fy1,Fy2 and Fy3.

26. A computer program of searching for a pair of fragments (Fx and Fy) from two biological sequences (X and Y), wherein a pattern of Fx and Fy satisfies a predetermined criterion, said computer program comprises:
(26.1) a code segment that selects a first fragment (Fx1) of X and a first fragment (Fy1) of Y, wherein a first pattern (p1) of Fx1 and Fy1 satisfies a first predetermined condition (c1);
(26.2) a code segment that extends a second fragment (Fx2) from Fx1 in a direction and extending a second fragment (Fy2) from Fy1 in the direction;
(26.3) a code segment that identifies a second pattern (p2) of Fx2 and Fy2; and
(26.4) a code segment that obtains the pair of fragments (Fx and Fy) based on p2 by a predetermined code segment.

27. The computer program of claim 26, wherein said predetermined code segment comprises:
(27.1) a code segment that determines if p2 satisfies a second predetermined condition (c2);
(27.1.1) a code segment that extends a third fragment (Fx3) from Fx2 in the direction and extending a third fragment (Fy3) from Fy2 in the direction if p2 satisfies c2; and
(27.1.2) a code segment that matches Fx2 and Fy2 if p2 does not satisfy c2.

28. The computer program of claim 27, wherein the code segment (27.1.1) comprises:
(28.1) a code segment that identifies a third pattern (p3) of Fx3 and Fy3;
(28.2) a code segment that determines if p3 satisfies a third predetermined condition (c3); and
(28.3) a code segment that obtains the pair of fragments (Fx and Fy) if p3 satisfies c3, wherein Fx includes Fx1, Fx2 and Fx3, and Fy includes Fy1,Fy2 and Fy3.

29. The computer program of claim 27, wherein said code segment (27.1.2) of matching Fx2 and Fy2 comprises
(29.1) a code segment that selects a subfragment (Fx2.1) in FX2 and a subfragment (Fy2.1) in FY2, wherein a subpattem (p2.1) of Fx2.1 and Fy2.1 satisfies a predetermined subcondition (c2.1);
(29.2) a code segment that inserts a gap into a region between Fx2.1 and Fx1 to exclude a part of biological data in Fx2 therefrom, whereby Fx2 becomes an updated fragment (Fx2') which includes the gap, but the part of biological data in Fx2 is excluded;
(29.3) a code segment that identifies an updated p2 (p2') between Fx2' and Fy2;
(29.4) a code segment that determining if p2' satisfies a predetermined requirement (r);
(29.4.1) a code segment that extends a third fragment (Fx3) from Fx2' in the direction and extending a third fragment (Fy3) from Fy2 in the direction if p2' satisfies r, wherein Fx3 includes the part of biological data in Fx2; and
(29.4.2) a code segment that obtains the pair of fragments (Fx and Fy) if p2' does not satisfy r, wherein Fx includes Fx1 and Fx2', and Fy includes Fy1 and Fy2.

30. The computer program of claim 29, wherein the code segment (29.4.1) comprises :
(30.1) a code segment that identifies a third pattern (p3) of Fx3 and Fy3;
(30.2) a code segment that determines if p3 satisfies a third predetermined condition (c3); and
(30.3) a code segment that obtains the pair of fragments (Fx and Fy) if p3 satisfies c3, wherein Fx includes Fx1, Fx2' and Fx3, and Fy includes Fy1,Fy2 and Fy3.
